# EUROPÄISCHE PATENTSCHRIFT

(11) **EP 1 940 765 B1**
(45) Veröffentlichungstag und Bekanntmachung des Hinweises auf die Patenterteilung: **13.05.2009**
(21) Anmeldenummer: 06807323.8
(22) Anmeldetag: 16.10.2006
(51) Int. Cl.: C07C 45/62, C07C 47/228

(54) **VERFAHREN ZUR KONTINUIERLICHEN HYDRIERUNG ODER HYDRIERENDEN AMINIERUNG**
METHOD FOR CONTINUOUS HYDROGENATION OR HYDROGENATING AMINATION
PROCEDE POUR UNE HYDROGENATION CONTINUE OU UNE AMINATION POUVANT ETRE HYDROGENEE

(30) Priorität: 17.10.2005 DE 102005049568
(43) Veröffentlichungstag der Anmeldung: 09.07.2008
(73) Patentinhaber: BASF SE, 67056 Ludwigshafen (DE)
(72) Erfinder: HOFFER, Bram, Willem, 69120 Heidelberg (DE); VOSS, Hartwig, 67227 Frankenthal (DE); SCHWAB, Ekkehard, 67434 Neustadt (DE); RHEUDE, Udo, 67166 Otterstadt (DE); KAIBEL, Gerd, 68623 Lampertheim (DE); HAAKE, Mathias, 68161 Mannheim (DE); EBERHARDT, Jan, 68167 Mannheim (DE)
(74) Vertreter: Reitstötter - Kinzebach
(86) Internationale Anmeldenummer: PCT/EP2006/067470
(87) Internationale Veröffentlichungsnummer: WO 2007/045641

(56) Entgegenhaltungen:
- EP-A1- 0 947 493
- DE-C1- 19 625 189

## Beschreibung

Die vorliegende Erfindung betrifft ein Verfahren zur kontinuierlichen Hydrierung ungesättigter Verbindungen bzw. zur kontinuierlichen hydrierenden Aminierung von Carbonylverbindungen.

Katalytische Hydrierungen an heterogenen Katalysatoren werden vielfach unter Einsatz von Festbettreaktoren durchgeführt, um die Vorzüge einer kontinuierlichen Verfahrensführung zu erhalten. Für verschiedene Reaktionen sind Festbettreaktoren jedoch ungeeignet, da sie für eine weitgehend vollständige Umsetzung nicht mehr handhabbare Dimensionen aufweisen müssten. Die Wärmemengen, die bei der Hydrierung ungesättigter Verbindungen freigesetzt werden, können bei geradem Durchgang durch einen Festbettreaktor Probleme hinsichtlich der Wärmeabfuhr hervorrufen. Somit muss zur Verringerung der anfallenden Wärmemengen gegebenenfalls der Umsatz begrenzt werden und/oder der Reaktoraustrag teilweise zurückgeführt werden, was aber zu einer unerwünschten Rückvermischung führt. Bei bestimmten Hydrierungen müssen außerdem speziell präparierte Katalysatoren hergestellt und eingesetzt werden, die bei Verlust der Aktivität - oft bereits nach kurzer Standzeit - ausgetauscht oder regeneriert werden müssen. Beim Festbettreaktor kann ein Katalysatorwechsel nicht im laufenden Betrieb erfolgen; der Katalysatorwechsel ist in der Regel nicht nur mit der Abstellung der Hydrieranlage, sondern auch der nachfolgenden Aufarbeitungsstufen verbunden.

Alternativ kann eine heterogen katalysierte Hydrierung in Form einer Suspensionsreaktion durchgeführt werden, wobei der Hydrierkatalysator durch Zufuhr mechanischer Energie z. B. in einem Rührkessel in einer Flüssigphase suspendiert wird, vgl. z.B. Ullmanns Enzyklopädie der technischen Chemie, 4. Aufl. Band 13, 1997, S. 138, Verlag Chemie Weinheim. Kontinuierlich durchströmte Rührkessel (CSTR) können aufgrund eines hohen Rückvermischungsgrades keine vollständige Umsetzung der Reaktanden erreichen, da der Umsetzungsgrad eine Funktion der Verweilzeit im Reaktor und der Reaktionsgeschwindigkeit ist. Bei vielen Anwendungen ist jedoch ein hoher Umsetzungsgrad der Reaktanden erwünscht, insbesondere bei der Herstellung von Riech- und Aromastoffen oder Arznei- und Pflanzenschutzmitteln bzw. deren Vorstufen.

Die EP-A- 0947493 offenbart ein Verfahren zur selektiven Flüssigphasenhydrierung von α,β-ungesättigten Carbonylverbindungen in einem kontinuierlich betriebenen Blasensäulenreaktor. Die Nachteile dieses Verfahrens liegen jedoch in einer nicht vollständigen Umsetzung.

Im Stand der Technik sind kaskadenförmige Anordnungen von kontinuierlich durchströmten Rührkesseln vorgeschlagen worden. Diese Anordnungen haben jedoch den Nachteil, dass sie einen hohen finanziellen Investitionsbedarf benötigen und insbesondere große Reaktorvolumen erfordern.

Die DE 196 25 189 C1 offenbart ein Verfahren zur katalytischen Hydrierung von Butindiol zu Butandiol nach einem Zweistufenverfahren. In diesem Verfahren wird in einer ersten Hydrierstufe das Butindiol in einem Rührreaktor hydriert, an den sich eine Hydrierung in einem Festbettreaktor anschließt. Durch dieses Verfahren soll die Nebenproduktbildung schwersiedender Komponenten verringert werden. Der Druckschrift ist nicht zu entnehmen, ob die Abtrennung des Katalysators kontinuierlich erfolgt.

Die EP-A 1 676 829, die nach dem Prioritätstag der vorliegenden Anmeldung veröffentlicht wurde, offenbart die Herstellung von alicyclischen Carbonsäuren oder deren Derivaten durch Selektivhydrierung der entsprechenden aromatischen Carbonsäure(-derivate) in mindestens zwei hintereinandergeschalteten Reaktoren, wobei mindestens ein Reaktor in Schlaufenfahrweise betrieben wird.

Der vorliegenden Erfindung liegt die Aufgabe zugrunde, ein Verfahren zur Hydrierung ungesättigter Verbindungen zur Verfügung zu stellen, das die Vorteile einer hohen Raum-Zeit-Ausbeute und eines hohen Umsatzes mit einer verbesserten Verfahrensflexibilität und einem geringen Investitionsaufwand vereint.

Die Aufgabe wird gelöst durch ein Verfahren zur kontinuierlichen Hydrierung ungesättigter Verbindungen, bei dem man
a) Teilchen eines ersten Hydrierkatalysators in einer Flüssigphase suspendiert, in der eine ungesättigte Verbindung gelöst ist,
b) die Flüssigphase in Gegenwart eines wasserstoffhaltigen Gases bei einem ersten Wasserstoffpartialdruck und bei einer ersten Temperatur im Gleichstrom entgegen der Richtung der Schwerkraft durch einen gepackten Blasensäulenreaktor führt,
c) den Austrag aus dem Blasensäulenreaktor einer Gas-Flüssig-Trennung zuführt,
d) die Flüssigphase aus Schritt c) einer Querstromfiltration zuführt, wobei man ein Retentat und ein Filtrat erhält,
e) das Retentat in den Schritt b) zurückführt
f) das Filtrat in Gegenwart eines wasserstoffhaltigen Gases bei einem zweiten Wasserstoffpartialdruck und bei einer zweiten Temperatur über ein Bett eines zweiten Hydrierkatalysators leitet.

Vorzugsweise ist der zweite Wasserstoffpartialdruck um wenigstens 10 bar höher als der erste Wasserstoffpartialdruck.

Das Verhältnis des Volumenstroms des Filtrats zum Volumenstrom des Retentats beträgt vorzugsweise 1:2000 bis 1:10, insbesondere 1:500 bis 1:10. Der Umsetzungsgrad im Schritt b) beträgt im Allgemeinen 80 bis 99,8%, vorzugsweise 90 bis 99,6%, bezogen auf die zugeführte ungesättigte Verbindung. Der Umsetzungsgrad im Schritt f) beträgt im Allgemeinen wenigstens 90%, bezogen auf die im Filtrat enthaltene ungesättigte Verbindung, insbesondere 95 bis 100 %.

Insbesondere bei der selektiven Hydrierung von Duft- und Aromastoff-Vorstufen hat sich das erfindungsgemäße Hydrierverfahren als besonders vorteilhaft erwiesen. Das erfindungsgemäße Verfahren ermöglicht einen extrem hohen Umsatz (Tiefhydrierung), was gerade im Duftstoff- und Aromabereich von Bedeutung ist, da hier bereits kleinste Verunreinigungen, beispielsweise aus nicht umgesetzten ungesättigten Vorstufen, den Geruchs- oder Geschmackseindruck empfindlich stören können. Die Tiefhydrierung ist in vielen Fällen geeignet, störende Verfärbungen zu beseitigen. Bei den farbgebenden Bestandteilen handelt es sich meist um (mehrfach) ungesättigte Verbindungen, die durch Hydrierung in farblose Verbindungen umgewandelt werden. Das erfindungsgemäße Hydrierverfahren kann weiterhin besonders vorteilhaft im Pflanzenschutz- und Pharmabereich eingesetzt werden, da dort angesichts der Einbringung von Substanzen in die Umwelt bzw. in den menschlichen oder tierischen Körper besonders hohe Anforderungen hinsichtlich vollständiger Umsetzungen und dadurch erzielbarer Produktreinheit gestellt werden.

Ungesättigte Verbindungen im Rahmen der vorliegenden Erfindung sind Verbindungen mit Mehrfachbindungen, an die Wasserstoff addiert werden kann. Vorzugsweise handelt es um Verbindungen mit wenigstens einer ethylenisch ungesättigten C=C-Doppelbindung und/oder C≡C-Dreifachbindung. Mehrere ethylenisch ungesättigte C=C-Doppelbindungen können isoliert, kumuliert und/oder konjugiert vorliegen.

Darüber hinaus zählen zu den ungesättigten Verbindungen auch solche, die wenigstens eine Kohlenstoff-Heteroatom-Doppelbindung, insbesondere Kohlenstoff-Sauerstoff-, Kohlenstoff-Stickstoff-, Kohlenstoff-Schwefel-, Kohlenstoff-Phosphor-, Kohlenstoff-Silicium- Doppelbindungen oder Kohlenstoff-Stickstoff- oder Kohlenstoff-Phosphor-Dreifachbindungen enthalten. Es können auch Doppel- oder Dreifachbindungssysteme zwischen Heteroatomen mit dem erfindungsgemäßen Verfahren hydriert werden. Insbesondere zählen zu den ungesättigten Verbindungen organische Moleküle, die ethylenische Doppelbindungen, Amid-, Carboxyl-, Nitril-, Imin-, Nitro-, Keto- und/oder, Aldehydgruppen aufweisen.

Eine bevorzugte Ausführungsform betrifft die selektive Hydrierung der C=C-Doppelbindung (unter Erhalt der C=O-Doppelbindung) von α,β-ungesättigte Carbonylverbindungen. Hierzu werden der erste und der zweite Hydrierkatalysator so ausgewählt, dass sie zur präferentiellen Hydrierung von Kohlenstoff-Kohlenstoff-Doppelbindungen vor Kohlenstoff-Sauerstoff-Doppelbindungen fähig sind.

α,β-ungesättigte Carbonylverbindungen sind insbesondere solche der nachstehenden Formel (I) 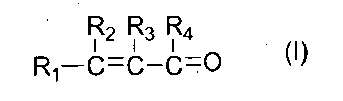 in der R₁ für Wasserstoff oder einen organischen Rest steht, vorzugsweise für Alkyl (z. B. C₁₋₂₀-Alkyl), Alkenyl (z. B. C₂₋₂₀-Alkenyl), Aryl (z. B. Phenyl) oder Aralkyl (z. B. Phenyl-C₁₋₆-alkyl), und R₂, R₃ und R₄ unabhängig voneinander für Wasserstoff oder C₁-₄-Alkyl stehen. Die Arylreste können bis zu 1, 2, 3 oder 4 Substituenten, wie C₁-₉-Alkyl oder C₁-₉-Alkoxy, aufweisen.

Besonders bevorzugte Einsatzstoffe sind Zimtaldehyd und Zimtaldehydderivate der folgenden Formel (II) 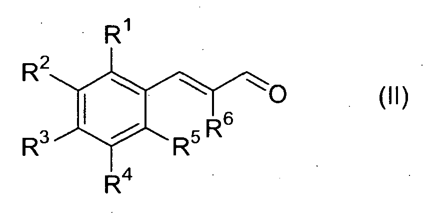 in der R¹, R², R³, R⁴ und R⁵ unabhängig voneinander für Wasserstoff, C₁-₉-Alkyl oder C₁-₉-Alkoxy stehen und R⁶ für Wasserstoff oder Methyl steht.

Ein bevorzugtes Zimtaldehydderivat ist Dehydrolysmeral (2-Methyl-3-(p-tert-butylphenyl)-propenal).

Bevorzugte Einsatzstoffe sind weiterhin Terpenaldehyde wie Citral; und Ketone mit terpenoidem Gerüst wie Pseudojonon (6,10-Dimethylundeca-3,5,9-trien-2-on).

Bevorzugte ungesättigte Verbindung sind außerdem Enamine, die man vorzugsweise in situ aus einer Carbonylverbindung und einem primären oder sekundären Amin bildet. D. h. das erfindungsgemäße Verfahren dient zur hydrierenden Aminierung von Carbonylverbindungen.

Geeignete Carbonylverbindungen, die der hydrierenden Aminierung unterworfen werden können, sind beispielsweise Acetaldehyd, Propionaldehyd, n-Butyraldehyd, iso-Butyraldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxyphenyl)acetaldehyd, (3, 4-Dimethoxyphenyl)acetaldehyd, Furfural, 4-Formyltetrahydropyran, 3-Formyltetrahydrofuran, 5-Formylvaleronitril, hydroformyliertes Polyisobuten (Polyisobutenaldehyd, PIBA), durch Metathese von 1-Penten und Cyclopenten erhaltenes und hydroformyliertes Oligomer, Aceton, 2-Butanon, 3-Methylbutan-2-on, 4-Methylpentan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Benzophenon, Cyclobutanon, Cyclopentanon, Cyclopentenon, Cyclohexanon, Cyclohexenon, 2,6-Dimethylcyclohexanon.

Eine besonders bevorzugte Carbonylverbindung ist 2-Methyl-3-(p-tert-butyl-phenyl)-propanal (Lysmeral).

Beispielsweise werden die folgenden primären oder sekundären Amine verwendet: Methylamin, Dimethylamin, Ethylamin, Diethylamin, n-Propylamin, Di-n-propyl-amin, iso-Propylamin, Di-isopropylamin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin, cis-2,6-Dimethylmorpholin und Pyrrolidin.

Das Amin wird bezüglich der zu aminierenden Carbonylgruppe in wenigstens stöchiometrischen Mengen eingesetzt. Im allgemeinen verwendet man 1,5 bis 10 Moläquivalente Amin pro Mol umzusetzender Carbonylgruppe.

Mit dem erfindungsgemäßen Verfahren besonders bevorzugt hergestellte Amine sind zum Beispiel N,N-Di(C₁₋₄-alkyl)cyclohexylamin (aus Cyclohexanon und Di(C₁₋₄-alkyl)-amin), n-Propylamine (wie Dimethylpropylamin) (aus Propionaldehyd und Dimethylamin), N,N-Dimethyl-N-isopropylamin (aus Aceton und Dimethylamin), N,N-Dimethyl-N-butylamine (aus Butanal, i-Butanal oder Butanon und Dimethylamin), N-Ethyl-N,N-diisopropylamin (aus Acetaldehyd und N,N-Diisopropylamin), Tris(2-Ethylhexyl)amin (aus 2-Ethylhexanal und Di(2-Ethylhexyl)amin) und Tributylamin (aus Butanal und Dibutylamin).

Eine besonders bevorzugte Ausführungsform des erfindungsgemäßen Verfahrens betrifft die Umsetzung von 2-Methyl-3-(p-tert-butyl-phenyl)-propanal mit cis-2,6-Dimethylmorpholin.

Eine weitere Anwendung des erfindungsgemäßen Verfahrens betrifft die selektive Hydrierung der Carbonylgruppe einer Verbindung, die über eine Carbonylgruppe, insbesondere eine Aldehydgruppe, und eine nicht damit konjugierte C=C-Doppelbindung verfügt, z. B. die Hydrierung von Citronellal zu Citronellol. Man verwendet hierzu Hydrierkatalysatoren, die zur präferentiellen Hydrierung von Kohlenstoff-Sauerstoff-Doppelbindungen vor Kohlenstoff-Kohlenstoff-Doppelbindungen fähig sind.

Sofern die zu hydrierende ungesättigte Verbindung (bzw. das Gemisch von Carbonylverbindung und Amin bei der hydrierenden Aminierung) und/oder das Hydrierungsprodukt davon unter den Hydrierbedingungen flüssig ist, kann die Flüssigphase im Wesentlichen frei sein von externen Lösungs- oder Verdünnungsmitteln sein. Als "externe Lösungs- oder Verdünnungsmittel" werden alle Lösungsmittel angesehen, die von der zu hydrierenden ungesättigten Verbindung oder der Carbonylverbindung und dem Amin bei der hydrierenden Aminierung oder dem jeweiligen Hydrierungsprodukt davon verschieden sind. Dies hat den Vorteil, dass das Entfernen eines Lösungsmittels nach Durchführung des Verfahrens nicht mehr erforderlich ist, um das gewünschte hydrierte Produkt zu erhalten.

Sofern mitverwendet, kommen als Verdünnungsmittel insbesondere Alkane, Cycloalkane, lineare oder cyclische aliphatische Mono-, Di-, Tri- oder Polyether, aliphatische Alkohole in Frage. Besonders geeignete Verdünnungsmittel sind C₁-C₆-Alkanole, besonders bevorzugt C₁-C₄-Alkanole, wie insbesondere Methanol.

Bei der Selektivhydrierung von α,β-ungesättigten Carbonylverbindungen kann der Zusatz einer katalytischen Menge von Ammoniak oder eines aliphatischen primären, sekundären oder tertiären Amins die Selektivität der Hydrierung verbessern. Tertiäre Amine, z.B. Tri(C₁-C₄-alkyl)amine, insbesondere Trimethylamin, sind besonders bevorzugt.

Als erster Hydrierkatalysator können handelsübliche Suspensionskatalysatoren verwendet werden. Insbesondere geeignet sind Katalysatoren, die Palladium, Rhodium, Ruthenium, Platin, Eisen, Cobalt, Kupfer, Nickel als hydrieraktive Metalle enthalten.

Häufig verwendet werden Raney-Katalysatoren. Der am leichtesten zugängliche und daher am meisten verwendete Raney-Katalysator ist Raney-Nickel.

Zur hydrierenden Aminierung oder zur präferentiellen Hydrierung von Kohlenstoff-Kohlenstoff-Doppelbindungen vor Kohlenstoff-Sauerstoff-Doppelbindungen eignen sich besonders solche Katalysatoren, die als Aktivkomponente mindestens Palladium enthalten. Neben Palladium kann der Katalysator auch weitere Aktivkomponenten, wie beispielsweise Zink, Cadmium, Platin, Silber oder ein Seltenerdmetall, wie beispielsweise Cer, enthalten. Der Katalysator kann in metallischer und/oder oxidischer Form eingesetzt werden. Vorzugsweise sind die Aktivkomponenten auf einem Trägermaterial aufgebracht. Als Trägermaterialien eignen sich beispielsweise SiO₂, TiO₂, ZrO₂, Al₂O₃ oder Kohlenstoff wie Graphite, Ruße oder Aktivkohle. Aktivkohle ist aufgrund ihrer leichten Suspendierbarkeit bevorzugt. Der Gehalt an Palladium beträgt vorzugsweise 0,1 bis 10 Gew.-%, insbesondere 0,5 bis 7 Gew.-% und besonders bevorzugt 2 bis 6 Gew.-%, bezogen auf das Gesamtgewicht des Katalysators.

Zur präferentiellen Hydrierung von Kohlenstoff-Sauerstoff-Doppelbindungen vor Kohlenstoff-Kohlenstoff-Doppelbindungen eignen sich besonders solche Katalysatoren, die als Aktivkomponente mindestens Ruthenium enthalten. Neben Ruthenium kann der Katalysator auch weitere Aktivkomponenten, wie beispielsweise Eisen, enthalten. Der Katalysator kann in metallischer und/oder oxidischer Form eingesetzt werden. Vorzugsweise sind die Aktivkomponenten auf einem Trägermaterial aufgebracht. Als Trägermaterialien eignen sich beispielsweise SiO₂, TiO₂, ZrO₂, Al₂O₃ oder Kohlenstoff wie Graphite, Ruße oder Aktivkohle. Aktivkohle ist aufgrund ihrer leichten Suspendierbarkeit bevorzugt. Der Gehalt an Ruthenium beträgt vorzugsweise 0,1 bis 10 Gew.-%, der Gehalt an Eisen vorzugsweise 0,1 bis 5 Gew.-%, insbesondere 0,5 bis 1,5 Gew.-%, bezogen auf das Gesamtgewicht des Katalysators.

Bevorzugt verwendet man Suspensionshydrierkatalysator-Teilchen mit einem mittleren Durchmesser von 0,0001 bis 2 mm, bevorzugt von 0,001 bis 1 mm, besonders bevorzugt von 0,005 bis 0,1 mm.

Das suspendierte Katalysatormaterial kann mit Hilfe gebräuchlicher Techniken in die Flüssigphase eingebracht und darin verteilt werden.

Als wasserstoffhaltiges Gas verwendet man in der Regel sowohl im Blasensäulenreaktor als auch im Festbettreaktor Wasserstoffgas mit einer Reinheit von z.B. wenigstens 99,5 Vol.-%. Die Menge des einzusetzenden Wasserstoffs ist in beiden Verfahrensstufen wenigstens äquimolar zur Anzahl der zu hydrierenden Doppelbindungsäquivalente der ungesättigten Verbindung in der Flüssigphase. Üblicherweise wird jedoch mit einem Überschuss von 1 bis 20 % gearbeitet.

Erfindungsgemäß führt man die Flüssigphase mit dem suspendierten ersten Hydrierkatalysator in Gegenwart des wasserstoffhaltigen Gases im Gleichstrom entgegen der Richtung der Schwerkraft durch einen gepackten Blasensäulenreaktor.

Der gepackte Blasensäulenreaktor besteht im Wesentlichen aus einem schlanken, senkrecht angeordneten zylindrischen Behälter, in den im unteren Teil eine Begasungsvorrichtung eingebaut ist. Als Gasverteiler werden z. B. poröse Platten, Lochböden, gelochte Rohre, Düsen oder Düsenböden verwendet. Die Gasphase wird kontinuierlich zugeführt. Weiterhin weist die erfindungsgemäß eingesetzte gepackte Blasensäule Packungselemente auf.

Die Packung bewirkt eine höhere Relativgeschwindigkeit der Flüssigphase gegenüber den Katalysatorteilchen, weil der Transport der Katalysatorteilchen durch die Packung in dem Reaktor, gehemmt wird, d.h. die Partikel werden gegenüber der umgebenden Flüssigkeit stärker zurückgehalten. In Verbindung mit der hohen volumenbezogenen Oberfläche der suspendierten Partikel wird die Raum-Zeit-Ausbeuten erhöht.

Bei der den Transport der Katalysatorteilchen hemmenden Packung handelt es sich üblicherweise um Einbauten in dem Reaktor, die so angeordnet sind, dass das Reaktionsgemisch beim Passieren des Reaktors durch die Packung gezwängt wird, d.h. die Einbauten füllen in der Regel den gesamten freien Querschnitt des Reaktors. Die Einbauten erstrecken sich vorzugsweise, aber nicht notwendigerweise über die gesamte Ausdehnung des Reaktors in Strömungsrichtung der Flüssigphase.

Die den Transport der Katalysatorteilchen hemmende Packung weist vorzugsweise Öffnungen oder Kanäle auf, deren hydraulischer Durchmesser das 2- bis 2000-fache, insbesondere das 5- bis 500-fache, besonders bevorzugt das 5- bis 100-fache des mittleren Durchmessers der Katalysatorteilchen beträgt.

Der hydraulische Durchmesser ist eine dem Fachmann geläufige Kenngröße zur Beschreibung des Äquivalentdurchmessers nichtkreisrunder Kanalstrukturen. Der hydraulische Durchmesser einer Öffnung ist als Quotient des 4-fachen Querschnitts der Öffnung und deren Umfang definiert. Bei Kanälen mit einem Querschnitt in Gestalt eines gleichschenkeligen Dreiecks lässt sich der hydraulische Durchmesser als 2bh/(b+2s) beschreiben, worin b für die Basis, h für die Höhe und s für die Schenkellänge des Dreiecks steht.

Die Öffnungen oder Kanäle geeigneter Packungen weisen vorteilhafterweise einen hydraulischen Durchmesser von 0, 5 bis 20 mm, vorzugsweise 1 bis 10 mm, besonders bevorzugt 1 bis 3 mm, auf.

Geeignete Packungselemente sind grundsätzlich, d.h. ihrer geometrischen Form nach, bereits aus der Destillations- und Extraktionstechnik bekannt. Für Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- und Extraktionstechnik.

Als Packungselemente eignen sich insbesondere Metallgewebepackungen bzw. Drahtgewebepackungen, z.B. der Bauart Montz A3, Sulzer BX, DX und EX. Anstelle von Metallgewebepackungen können auch Packungen aus anderen gewebten, gewirkten oder gefilzten Materialien verwendet werden. Weiterhin eignen sich Packungen ebener oder gewellter Bleche, bevorzugt ohne Perforation oder andere größere Öffnungen, beispielsweise entsprechend den Bauarten Montz B1 oder Sulzer Mellapak. Vorteilhaft sind auch Packungen aus Streckmetall, wie z.B. Packungen des Typs Montz BSH.

Beim erfindungsgemäßen Verfahren wird die Hydrierung in dem Blasensäulenreaktor als auch in dem Festbettreaktor kontinuierlich betrieben.

Die erste Hydrierstufe in dem gepackten Blasensäulenreaktor erfolgt im Allgemeinen bei einem Wasserstoffpartialdruck von 1 bis 100 bar, vorzugsweise 5 bis 25 bar.

Die Temperaturen in dem Blasensäulenreaktor beträgt üblicherweise 25 bis 150 °C, vorzugsweise 50 bis 100 °C.

Die Leerrohrgeschwindigkeit, mit der die Flüssigphase durch den gepackten Blasensäulenreaktor geführt wird, beträgt vorzugsweise mindestens 100 m³/m²h, vorzugsweise 100 bis 500 m³/m²h, insbesondere 150 bis 300 m³/m²h, die der Gasphase mehr als 5 Nm³/m²h, insbesondere 10 bis 200 Nm³/m²h. Um ausreichend hohe Leerrohrgeschwindigkeiten zu erzielen, wird das Retentat aus Schritt d) zurückgeführt.

Das erfindungsgemäße Verfahren sieht zur Abtrennung des suspendierten esten Hydrierkatalysators eine Querstromfiltration vor. Bei der Querstromfiltration wird das zu filtrierende Medium tangential zu Oberfläche einer Membran bewegt, die Flüssigkeit hindurchtreten lässt, den suspendierten Katalysator aber zurück hält. Der Volumenstrom des Filtrats (Permeats) verläuft dabei senkrecht zur Flussrichtung des Mediums. Die treibende Kraft des Prozesses ist der Transmembrandruck. Bei der Querstromfiltration verhindert die Strömung parallel zur Filteroberfläche den Aufbau eines Filterkuchens.

Die für das erfindungsgemäße Verfahren eingesetzten Filtermembranen haben, abhängig von der Partikelgröße des eingesetzten katalysators, vorzugsweise Porendurchmesser im Bereich von 0,5 nm bis 20 µm, insbesondere im Bereich von 1 nm bis 10 µm und am meisten bevorzugt von 2 nm bis 1 µm.

Die Trennschichten der Filtermembranan können aus organischen Polymeren (wie Polytetrafluorethylen, Polyvinylidenfluorid, Polysulfon, Polyethersulfon, Polyetheretherketon, Polyamid), Keramik (wie α-Al₂O₃, ZrO₂, TiO₂, SiC oder gemischte keramische Werkstoffe), Metall, Kohlenstoff oder Kombinationen daraus bestehen und müssen in dem Reaktionsmedium bei Reaktionstemperatur stabil sein. Aus mechanischen Gründen sind die Trennschichten in der Regel auf einer gröberen ein- oder mehrschichtigen porösen Unterstruktur aufgebracht, die aus dem gleichen oder aus einem anderen Material als die Trennschicht besteht. Beispiele sind metallische Trennschichten auf einer Unterstruktur aus Metall; keramische Trennschichten auf einer Unterstruktur aus Metall, Keramik oder Kohlenstoff; polymere Trennschichten auf einer Unterstruktur aus Polymer, Metall, Keramik oder Keramik auf Metall; oder Kohlestoff-Trennschichten auf einer Unterstruktur aus Kohlenstoff ,Metall oder Keramik.

Die Membranen werden üblicherweise in druckfeste Gehäuse eingesetzt, welche die Trennung zwischen katalysatorhaltigem Retentat und katalysatorfreiem Filtrat bei den für die Filtration erforderlichen Druckbedingungen erlauben. Die Membranen können in Flach-, Scheiben-, Rohr-, Multikanalelement-, Kapillar- oder Wickelgeometrie ausgeführt sein, für die entsprechende Druckgehäuse verfügbar sind, die eine Trennung zwischen Retentat und Filtrat erlauben. Je nach Flächenbedarf kann ein Filterelement mehrere Kanäle enthalten. Weiterhin können mehrere dieser Elemente in einem Gehäuse zu einem Modul zusammengefasst werden. In einer bevorzugten Ausführungsform werden Metallmembranen verwendet, die z. B. mit den Gehäusen verschweißt oder als Scheiben ausgeführt sind.

Das Verfahren wird vorzugsweise so betrieben, dass sich auf der Retentatseite der Membran eine möglichst dünne Deckschicht aus Katalysator ausbildet. Daher wird die katalysatorhaltige Flüssigphase vorzugsweise mit hoher Geschwindigkeit durch die Moduln geleitet, um eine ausreichende Scherung an der Membranoberfläche zu erzeugen. Alternativ kann eine Scherung auch durch Bewegen der Membran oder durch Rührelemente zwischen den Membranen erzeugt werden (Rotations- oder Vibrationsmodule).

Störende Deckschichten können durch kurzzeitige Strömungsumkehr zwischen Retentat- und Filtratseite beseitigt werden. Hierzu hebt man den Druck auf der Filtratseite über den Druck auf der Retentatseite an.

Der optimale Transmembrandruck wird von Faktoren, wie dem Durchmesser der Membranporen, den hydrodynamischen Bedingungen, die den Deckschichtaufbau beeinflussen, und der mechanishen Stabilität der Membran bei der Betriebstemperatur beeinflusst. Er liegt meist bei mindestens 0,1 bar, insbesondere 0,2 bis 50 bar, vorzugsweise 0,5 bis 25 bar. Höhere Transmembrandrücke führen zumeist zu höheren Permeatflüssen. Die erreichbaren Permeatflüsse sind stark von der eingesetzten Membranart und -geometrie, von den Prozessbedingungen, von der Zusammensetzung der katalysatorhaltigen Flüssigphase, der Katalysatorkonzentration und der Art des Katalysators abhängig. Die Flüsse liegen üblicherweise zwischen 1 und 2000 kg/m²/h. Die erreichbare Katalysatorrückhaltung beträgt mehr als 99 %.

Erfindungsgemäß wird das Filtrat einer weiteren Hydrierungsstufe an einem Bett eines zweiten Hydrierkatalysators zugeführt. Die Umsetzung am zweiten Hydrierkatalysator erfolgt in Allgemeinen in einem Festbettreaktor.

Festbettreaktoren sind an sich bekannt. In einem Festbettreaktor ist der Hydrierkatalysator in Form einer Füllkörperschüttung in einem senkrecht stehenden zylindrischen Behälter mit ungeteiltem Querschnitt räumlich fixiert angeordnet. Über dieses Katalysatorbett werden die Flüssigphase und das wasserstoffhaltige Gas geleitet, wobei die flüssige Phase sowohl von oben als auch von unten durchgesetzt und die Gasphase im Gleich- oder im Gegenstrom dazu geführt werden kann.

Bei der sogenannten Sumpffahrweise wird das Filtrat entgegen der Schwerkraft durch das Bett des Katalysators geführt. Bei dieser so genannten Sumpffahrweise kann es zu einer Bettausdehnung kommen.

In einer bevorzugten Ausführungsform gibt man jedoch das Filtrat am Kopf des Festbettreaktors auf und lässt es unter dem Einfluss der Schwerkraft über das Bett des zweiten Hydrierkatalysators rieseln.

Vorzugsweise werden sowohl die Flüssigphase als auch die wasserstoffhaltige Gasphase kontinuierlich eingespeist, wobei für beide Phasen eine teilweise Rückführung möglich ist.

Als zweiter Hydrierkatalysator eignen sich grundsätzlich auch die bereits beim ersten Hydrierkatalysator aufgeführten Katalysatormaterialien. Somit sind insbesondere geeignet Katalysatoren, die Palladium, Rhodium, Ruthenium, Platin, Eisen, Cobalt, Kupfer, Nickel als hydrieraktive Metalle enthalten.

Zur hydrierenden Aminierung oder zur präferentiellen Hydrierung von Kohlenstoff-Kohlenstoff-Doppelbindungen vor Kohlenstoff-Sauerstoff-Doppelbindungen eignen sich besonders solche Katalysatoren, die als Aktivkomponente mindestens Palladium enthalten. Neben Palladium kann der Katalysator auch weitere Aktivkomponenten, wie beispielsweise Zink, Cadmium, Platin, Silber oder ein Seltenerdmetall, wie beispielsweise Cer, enthalten. Der Katalysator kann in metallischer und/oder oxidischer Form eingesetzt werden. Vorzugsweise sind die Aktivkomponenten auf einem Trägermaterial aufgebracht.

Bevorzugt umfassen die Teilchen des zweiten Hydrierkatalysators ein Trägermaterial ausreichender mechanischer Stabilität, wie z. B. Oxide von Silicium, Aluminium, Titan und/oder Zirkonium. Ein bevorzugtes Trägermaterial ist Aluminiumoxid. Besonders bevorzugt wird ein auf Aluminiumoxid geträgerter Palladiumkatalysator verwendet.

Die Festbetthydrierkatalysatoren werden üblicherweise als stückiges Material oder als Presslinge in Zylinder-, Tabletten- oder auch Kugelform verwendet. Die Strangpresslinge haben gewöhnlich Durchmesser von 1,5 bis 3,5 mm und Längen bis zu 20 mm. Die Abmessungen von Tabletten oder stückigem Material liegen üblicherweise zwischen 2 und 8 mm.

Die Hydrierung im Festbettreaktor erfolgt vorzugsweise bei einer Temperatur, die um wenigstens 10 °C höher ist als die Temperatur im Blasensäulenreaktor, insbesondere um wenigstens 40 °C höher. Die Hydrierung in dem Festbettreaktor erfolgt vorzugsweise bei Reaktionstemperaturen von 50 bis 250 °C, vorzugsweise von 60 bis 200 °C und insbesondere von 75 bis 160 °C.

Der Wasserstoffpartialdruck im Festbettreaktor beträgt im Allgemeinen 15 bis 50 bar, vorzugsweise 20 bis 100 bar.

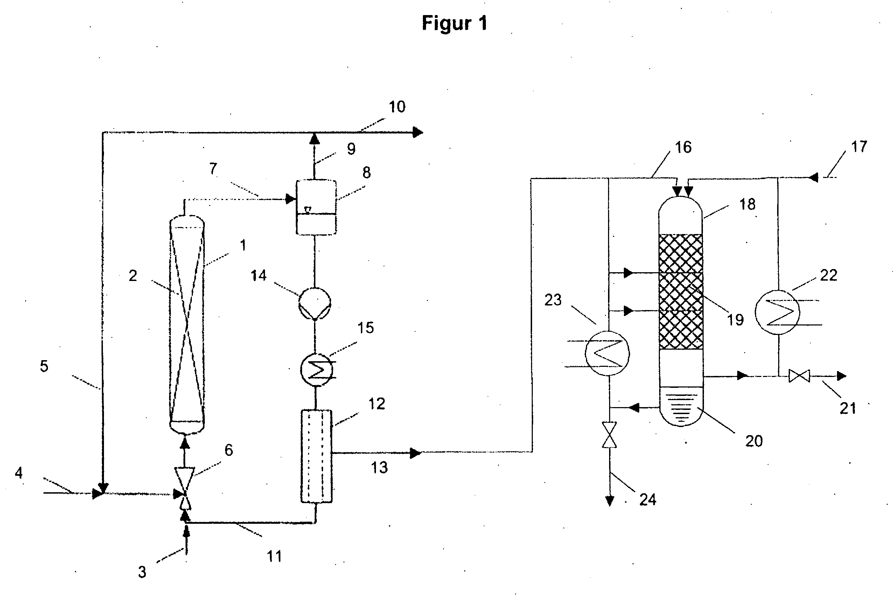

Das erfindungsgemäße Verfahren wird durch die beigefügte Figur 1 und die nachstehenden Beispiele näher veranschaulicht.

Figur 1 zeigt schematisch eine zur Durchführung des erfindungsgemäßen Verfahrens geeignete Anlage mit einem Reaktor (Blasensäule) **1** mit einer Packung **2,** die den Transport der Katalysatorteilchen hemmt. In den Reaktor werden über die Leitungen **3** Flüssigkeit und über die Leitung **4** Wasserstoffgas eingeführt. Das Kreisgas **5** wird mittels der Mischdüse **6** mit Frischgas und der durch die Pumpe **14** im Kreis geführten Suspension **11** eingemischt. Der Reaktoraustrag wird über die Leitung **7** in das Abscheidegefäß **8** gefahren, in dem die Gasphase abgeschieden und über Leitung **9** abgeführt wird. Von dieser Gasmenge wird zur Begrenzung der Aufpegelung von gasförmigen Verunreinigungen ein Teilstrom über die Leitung **10** entnommen und die verbleibende Restmenge über die Leitung **5** in den Reaktor geführt. Der suspendierte Katalysator verbleibt im Reaktorsystem, indem er über einen Querstromfilter **12** zurückgehalten und nur katalysatorfreie Flüssigphase über die Leitung **13** austritt und entnommen wird. Über den Wärmetauscher **15** kann die Temperatur im Suspensionsreaktorsystem gezielt eingestellt werden.

Das über die Leitung **13** entnommene Produkt wird über die Leitung **16** dem Festbettreaktor **18** zugeführt. Dem Festbettreaktor wird zusätzlich über Leitung **17** Wasserstoff zugeführt. Die über die Leitung **16** zugeführte Flüssigphase wird am Kopf des Festbettreaktors **18** aufgegeben und rieselt unter dem Einfluss der Schwerkraft über die Festbetthydrierungskatalysator-Teilchen **19.** Am Ende der Katalysatorschicht werden die flüssige und die gasförmige Phase entweder bei Reaktionstemperatur in einem Heißabscheider **20** oder nach Durchlaufen eines Kühlers in einem Kaltabscheider getrennt. Die Gasphase wird über die Leitung **21** abgeführt und das tiefhydrierte Produkt kann über Leitung **24** entnommen werden. Über die Wärmetauscher **22** und **23** kann die Temperatur im Festbettreaktorsystem gezielt eingestellt werden.

### Beispiel 1: Hydrierende Aminierung von Lysmeral (2-Methyl-3-(p-tert-butyl-phenyl)-propanal) mit cis-2,6-Dimethylmorpholin zu Fenpropimorph

Man verwendete eine Anlage wie in Fig. 1 dargestellt, die eine mit einer Drahtgewebepackung üblicher Webart (Kanthal) bestückte Blasensäule (500 mm Länge, 20 mm Durchmesser) umfasste. Die Drahtstärke betrug 0,1 mm, die Knickbreite 1 mm, der Neigungswinkel der Knicke gegen die Vertikale war 60°. Die volumenbezogene Oberfläche der Packung betrug 2000 m²/m³ (bezogen auf die geometrische Oberfläche der Gewebe, d. h. eine theoretische Oberfläche, bei der die Gewebe als glatte Flächen gedacht sind).

Als Zulauf diente ein Gemisch aus Lysmeral und cis-2,6-Dimethylmorpholin in einem Molverhältnis von 1:2,5. Die Zulaufmenge war 100 g/h.

In dem Zulauf wurde ein Palladium-Kohle Suspensionshydrierungskatalysator suspendiert, der 5 % Palladium auf Aktivkohle enthielt. Die Reaktion erfolgte kontinuierlich bei 80 °C und 10 bar Wasserstoffdruck. Die Flüssigkeit mit dem suspendierten Katalysator und das Gas wurden mit einer Geschwindigkeit von 200 m³/m² h im Gleichstrom entgegen der Richtung der Schwerkraft durch den Reaktor geführt. Der Umsatz betrug 99,5 %. Die Raumzeitausbeute betrug 425 kg_{FPM}/(m³h).

Die Querfiltrationseinheit umfasste eine Filterkerze (α-Al₂O₃ und ZrO₂ mit 100 nm Porenweite) in einem Querstromfiltergehäuse (d x h = 25 mm x 176 mm). Der Transmembrandruck war 0,3 bar.

Das Filtrat wurde als Einsatzstoff für den Nachreaktor verwendet (100 ml/h). Bei dem Reaktor (Durchmesser d= 27,3 mm, Bettlänge = 230 mm) handelt es sich um einen mit Palladium-Aluminiumoxid-Festbetthydrierungskatalysator-Teilchen gefüllten Festbettreaktor. Die Reaktion wurde bei 140 °C und 30 bar Wasserstoffdruck in Rieselfahrweise durchgeführt. Der Umsatz betrug 92,9 %, was einen Gesamtumsatz von 99,96 % bedeutet.

### Beispiel 2: Hydrierung von Dehydrolysmeral (2-Methyl-3-(p-tert-butyl-phenyl)-propenal) zu Lysmeral (2-Methyl-3-(p-tert-butyl-phenyl)-propanal)

Man verwendete eine Anlage wie in Beispiel 1 beschrieben, als Zulauf diente Dehydrolysmeral (30 Gew.-% in Methanol). In dem Zulauf wurde ein Palladium-Kohle Suspensionshydrierungskatalysator suspendiert, der 5 % Palladium auf Aktivkohle enthielt. Die Reaktion erfolgte kontinuierlich bei 60 °C und 10 bar Wasserstoffdruck. Die Flüssigkeit mit dem suspendierten Katalysator und das Gas wurden mit einer Geschwindigkeit von 200 m³/m² h im Gleichstrom entgegen der Richtung der Schwerkraft durch den Reaktor geführt. Der Umsatz betrug 94 %. Die Raumzeitausbeute betrug 0,3 L/(L h).

Das Produkt aus der gepackten Blasensäule wurde als Einsatzstoff für den Nachreaktor verwendet (200 ml/h). Bei dem Reaktor (Durchmesser d= 27,3 mm, Bettlänge = 400 mm) handelt es sich um einen mit Teilchen eines Palladium-Aluminiumoxid-Hydrierkatalysators gefüllten Festbettreaktor. Die Reaktion wurde bei 115 °C und 30 bar Wasserstoffdruck in Rieselfahrweise durchgeführt. Das restliche Dehydrolysmeral (etwa 1,0 bis 1,5% ) wurde vollständig (weit unter 0,1 %) umgewandelt.

## Patentansprüche

1. Verfahren zur kontinuierlichen Hydrierung ungesättigter Verbindungen, bei dem man
a) Teilchen eines ersten Hydrierkatalysators in einer Flüssigphase suspendiert, in der eine ungesättigte Verbindung gelöst ist,
b) die Flüssigphase in Gegenwart eines wasserstoffhaltigen Gases bei einem ersten Wasserstoffpartialdruck und bei einer ersten Temperatur im Gleichstrom entgegen der Richtung der Schwerkraft durch einen gepackten Blasensäulenreaktor führt,
c) den Austrag aus dem Blasensäulenreaktor einer Gas-Flüssig-Trennung zuführt,
d) die Flüssigphase aus Schritt c) einer Querfiltration zuführt, wobei man ein Retentat und ein Filtrat erhält,
e) das Retentat in den Schritt b) zurückführt
f) das Filtrat in Gegenwart eines wasserstoffhaltigen Gases bei einem zweiten Wasserstoffpartialdruck und bei einer zweiten Temperatur über ein Bett eines zweiten Hydrierkatalysators leitet,.

2. Verfahren nach Anspruch 1, wobei man im Schritt b) die Flüssigphase mit einer Leerrohrgeschwindigkeit von wenigstens 100 m³/m²h, vorzugsweise 100 bis 500 m³/m²h, insbesondere 150 bis 300 m³/m²h durch den gepackten Blasensäulenreaktor führt.

3. Verfahren nach Anspruch 1 oder 2, wobei man im Schritt f) das Filtrat am Kopf eines Festbettreaktors aufgibt und das Filtrat unter dem Einfluss der Schwerkraft über das Bett des zweiten Hydrierkatalysators rieselt.

4. Verfahren nach Anspruch 1 oder 2, wobei man im Schritt f) das Filtrat entgegen der Richtung der Schwerkraft durch das Bett des zweiten Hydrierkatalysators leitet.

5. Verfahren nach einem der vorhergehenden Ansprüche, wobei der zweite Wasserstoffpartialdruck um wenigstens 10 bar höher ist als der erste Wasserstoffpartialdruck.

6. Verfahren nach einem der vorhergehenden Ansprüche, wobei der erste Wasserstoffpartialdruck 1 bis 100 bar beträgt.

7. Verfahren nach einem der vorhergehenden Ansprüche, wobei der zweite Wasserstoffpartialdruck 15 bis 130 bar beträgt.

8. Verfahren nach einem der vorhergehenden Ansprüche, wobei die zweite Temperatur um wenigstens 10 °C höher ist als die erste Temperatur.

9. Verfahren nach einem der vorhergehenden Ansprüche, wobei die erste Temperatur 25 bis 150 °C, vorzugsweise von 50 bis 100 °C beträgt.

10. Verfahren nach einem der vorhergehenden Ansprüche, wobei die zweite Temperatur 50 bis 250 °C, vorzugsweise von 60 bis 200 °C und insbesondere von 75 bis 160 °C beträgt.

11. Verfahren nach einem der vorhergehenden Ansprüche, wobei es sich bei der ungesättigten Verbindung um eine α,β-ungesättigte Carbonylverbindung handelt und der erste und der zweite Hydrierkatalysator zur präferentiellen Hydrierung von Kohlenstoff-Kohlenstoff-Doppelbindungen vor Kohlenstoff-Sauerstoff-Doppelbindungen fähig sind.

12. Verfahren nach Anspruch 11, wobei die ungesättigte Verbindung ausgewählt ist unter Zimtaldehyd, Zimtaldehydderivaten, Terpenaldehyden und Ketonen mit terpenoidem Gerüst.

13. Verfahren nach Anspruch 12, wobei es sich bei dem Zimtaldehydderivat um 2-Methyl-3-(p-tert-butyl-phenyl)-propenal oder bei dem Terpenaldehyd um Citral oder bei dem Keton mit terpenoidem Gerüst um 6,10-Dimethyl-3,5,9-undecatrien-2-on handelt.

14. Verfahren nach einem der Ansprüche 1 bis 10, wobei es sich bei der ungesättigten Verbindung um ein Enamin handelt.

15. Verfahren nach Anspruch 14, wobei man das Enamin in situ aus einer Carbonylverbindung und einem primären oder sekundären Amin herstellt.

16. Verfahren nach Anspruch 15, wobei die Carbonylverbindung 2-Methyl-3-(p-tert-butyl-phenyl)-propanal ist.

17. Verfahren nach Anspruch 16, wobei das Amin cis-2,6-Dimethylmorpholin ist.

18. Verfahren nach einem der Ansprüche 13 bis 16, wobei die Flüssigphase im Wesentlichen kein externes Lösungs- oder Verdünnungsmittel umfasst.

19. Verfahren nach einem der Ansprüche 11 bis 13 , wobei die Flüssigphase ein externes Lösungs- oder Verdünnungsmittel umfasst.

20. Verfahren nach einem der vorhergehenden Ansprüche, wobei der erste Hydrierkatalysator Palladium auf einem Kohleträger umfasst.

21. Verfahren nach einem der vorhergehenden Ansprüche, wobei der zweite Hydrierkatalysator Palladium auf einem Aluminiumoxidträger umfasst.

## Claims

1. A process for continuously hydrogenating unsaturated compounds, in which
a) particles of a first hydrogenation catalyst are suspended in a liquid phase in which an unsaturated compound is dissolved,
b) the liquid phase, in the presence of a hydrogenous gas at a first partial hydrogen pressure and at a first temperature, is conducted through a packed bubble column reactor in cocurrent counter to the direction of gravity,
c) the effluent from the bubble column reactor is sent to a gas-liquid separation,
d) the liquid phase from step c) is sent to a crossfiltration to obtain a retentate and a filtrate,
e) the retentate is recycled into step b),
f) the filtrate, in the presence of a hydrogenous gas at a second partial hydrogen pressure and at a second temperature, is passed over a bed of a second hydrogenation catalyst.

2. The process according to claim 1, wherein the liquid phase is conducted through the packed bubble column reactor in step b) at a superficial velocity of at least 100 m³/m²h, preferably from 100 to 500 m³/m²h, in particular from 150 to 300 m³/m²h.

3. The process according to claim 1 or 2, wherein, in step f), the filtrate is introduced at the top of a fixed bed reactor and the filtrate trickles over the bed of the second hydrogenation catalyst under the influence of gravity.

4. The process according to claim 1 or 2, wherein, in step f), the filtrate is passed through the bed of the second hydrogenation catalyst counter to the direction of gravity.

5. The process according to any of the preceding claims, wherein the second partial hydrogen pressure is at least 10 bar higher than the first partial hydrogen pressure.

6. The process according to any of the preceding claims, wherein the first partial hydrogen pressure is from 1 to 100 bar.

7. The process according to any of the preceding claims, wherein the second partial hydrogen pressure is from 15 to 130 bar.

8. The process according to any of the preceding claims, wherein the second temperature is at least 10°C higher than the first temperature.

9. The process according to any of the preceding claims, wherein the first temperature is from 25 to 150°C, preferably from 50 to 100°C.

10. The process according to any of the preceding claims, wherein the second temperature is from 50 to 250°C, preferably from 60 to 200°C and in particular from 75 to 160°C.

11. The process according to any of the preceding claims, wherein the unsaturated compound is an α,β-unsaturated carbonyl compound and the first and the second hydrogenation catalyst are capable of preferential hydrogenation of carbon-carbon double bonds over carbon-oxygen double bonds.

12. The process according to claim 11, wherein the unsaturated compound is selected from cinnamaldehyde, cinnamaldehyde derivatives, terpene aldehydes and ketones with terpenoid skeleton.

13. The process according to claim 12, wherein the cinnamaldehyde derivative is 2-methyl-3-(p-tert-butylphenyl)propenal or the terpene aldehyde is citral or the ketone with terpenoid skeleton is 6,10-dimethyl-3,5,9-undecatrien-2-one.

14. The process according to any of claims 1 to 10, wherein the unsaturated compound is an enamine.

15. The process according to claim 14, wherein the enamine is prepared in situ from a carbonyl compound and a primary or secondary amine.

16. The process according to claim 15, wherein the carbonyl compound is 2-methyl-3-(p-tert-butylphenyl)propanal.

17. The process according to claim 16, wherein the amine is cis-2,6- dimethylmorpholine.

18. The process according to any of claims 13 to 16, wherein the liquid phase comprises substantially no external solvent or diluent.

19. The process according to any of claims 11 to 13, wherein the liquid phase comprises an external solvent or diluent.

20. The process according to any of the preceding claims, wherein the first hydrogenation catalyst comprises palladium on a carbon support.

21. The process according to any of the preceding claims, wherein the second hydrogenation catalyst comprises palladium on an alumina support.

## Revendications

1. Procédé pour l'hydrogénation continue de composés insaturés, dans lequel
a) on met en suspension dans une phase liquide des particules d'un premier catalyseur d'hydrogénation, dans lequel est dissous un composé insaturé,
b) on guide la phase liquide en présence d'un gaz contenant de l'hydrogène à une première pression partielle d'hydrogène et à une première température dans un flux parallèle dans le sens inverse de la gravité dans un réacteur à colonne à bulles garni,
c) on alimente le produit évacué du réacteur à colonne à bulles dans une séparation gaz-liquide,
d) on alimente la phase liquide de l'étape c) dans une filtration transversale, avec obtention d'un rétentat et d'un filtrat,
e) on recycle le rétentat dans l'étape b)
f) on guide le filtrat en présence d'un gaz contenant de l'hydrogène à une deuxième pression partielle d'hydrogène et à une deuxième température sur un lit d'un deuxième catalyseur d'hydrogénation.

2. Procédé selon la revendication 1, où on guide, dans l'étape b), la phase liquide à une vitesse de tube vide d'au moins 100 m³/m²h, de préférence de 100 à 500 m³/m²h, en particulier de 150 à 300 m³/m²h, dans le réacteur à colonne à bulles garni.

3. Procédé selon la revendication 1 ou 2, où on place, dans l'étape f), le filtrat en tête d'un réacteur à lit fixe et le filtrat ruisselle sous l'influence de la gravité sur le lit du deuxième catalyseur d'hydrogénation.

4. Procédé selon la revendication 1 ou 2, où on guide, dans l'étape f), le filtrat en sens inverse de la gravité au travers du lit du deuxième catalyseur d'hydrogénation.

5. Procédé selon l'une quelconque des revendications précédentes, où la deuxième pression partielle d'hydrogène est supérieure d'au moins 10 bars à la première pression partielle d'hydrogène.

6. Procédé selon l'une quelconque des revendications précédentes, où la première pression partielle d'hydrogène est de 1 à 100 bars.

7. Procédé selon l'une quelconque des revendications précédentes, où la deuxième pression partielle d'hydrogène est de 15 à 130 bars.

8. Procédé selon l'une quelconque des revendications précédentes, où la deuxième température est supérieure d'au moins 10°C à la première température.

9. Procédé selon l'une quelconque des revendications précédentes, où la première température est de 25 à 150°C, de préférence de 50 à 100°C.

10. Procédé selon l'une quelconque des revendications précédentes, où la deuxième température est de 50 à 250°C, de préférence de 60 à 200°C et en particulier de 75 à 160°C.

11. Procédé selon l'une quelconque des revendications précédentes, où il s'agit, pour le composé insaturé, d'un composé de type carbonyle α,β-insaturé et le premier et le deuxième catalyseur d'hydrogénation sont aptes à l'hydrogénation préférentielle de doubles liaisons carbone-carbone avant les doubles liaisons carbone-oxygène.

12. Procédé selon la revendication 11, où le composé insaturé est choisi parmi l'aldéhyde cinnamique, les dérivés de l'aldéhyde cinnamique, les aldéhydes terpéniques et les cétones avec une structure terpénoïde.

13. Procédé selon la revendication 12, où il s'agit, pour le dérivé de l'aldéhyde cinnamique, de 2-méthyl-3-(p-tert-butylphényl)-propénal ou, pour l'aldéhyde terpénique, de citral ou, pour la cétone avec une structure terpénoïde, de 6,10-diméthyl-3,5,9-undécatrién-2-one.

14. Procédé selon l'une quelconque des revendications 1 à 10, où il s'agit, pour le composé insaturé, d'une énamine.

15. Procédé selon la revendication 14, où on prépare l'énamine in situ à partir d'un composé carbonyle et d'une amine primaire ou secondaire.

16. Procédé selon la revendication 15, où le composé carbonyle est le 2-méthyl-3-(p-tert-butylphényl)-propanal.

17. Procédé selon la revendication 16, où l'amine est la cis-2,6-diméthylmorpholine.

18. Procédé selon l'une quelconque des revendications 13 à 16, où la phase liquide ne comprend essentiellement pas de solvant ni de diluant externe.

19. Procédé selon l'une quelconque des revendications 11 à 13, où la phase liquide comprend un solvant ou un diluant externe.

20. Procédé selon l'une quelconque des revendications précédentes, où le premier catalyseur d'hydrogénation comprend du palladium sur un support en carbone.

21. Procédé selon l'une quelconque des revendications précédentes, où le deuxième catalyseur d'hydrogénation comprend du palladium sur un support en oxyde d'aluminium.
